# EUROPÄISCHE PATENTSCHRIFT

(11) **EP 1 735 261 B1**
(45) Veröffentlichungstag und Bekanntmachung des Hinweises auf die Patenterteilung: **13.08.2008**
(21) Anmeldenummer: 05716479.0
(22) Anmeldetag: 31.03.2005
(51) Int. Cl.: C07C 67/055, C07C 69/15

(54) **VERFAHREN ZUR ETHYLEN-RÜCKGEWINNUNG IN EINEM KREISGASPROZESS ZUR HERSTELLUNG VON VINYLACETAT**
METHOD FOR RECOVERY OF ETHYLENE IN A RECIRCULATING GAS PROCESS FOR THE PRODUCTION OF VINYL ACETATE
PROCEDE DE RECUPERATION D'ETHYLENE DANS UN PROCESSUS A GAZ RECYCLE POUR PRODUIRE DE L'ACETATE DE VINYLE

(30) Priorität: 15.04.2004 DE 102004018284
(43) Veröffentlichungstag der Anmeldung: 27.12.2006
(73) Patentinhaber: Wacker Chemie AG, 81737 München (DE)
(72) Erfinder: DAFINGER, Willibald, 94133 Röhrnbach (DE); HOLL, Peter, 84547 Emmerting (DE); KAISER, Wilhelm, 84489 Burghausen (DE); GUBA, Jürgen, 84489 Burghausen (DE)
(74) Vertreter: Schuderer, Michael
(86) Internationale Anmeldenummer: PCT/EP2005/003388
(87) Internationale Veröffentlichungsnummer: WO 2005/100296

(56) Entgegenhaltungen:
- EP-A- 0 985 657
- WO-A-01/90042
- GB-A- 1 264 377
- US-A- 3 714 237
- US-A1- 2003 060 642

## Beschreibung

Die Erfindung betrifft ein Verfahren zur Ethylen-Rückgewinnung in einem Kreisgasprozess zur Herstellung von Vinylacetat, wobei ein Teilstrom des ethylenhaltigen Produktstroms ausgeschleust wird und einem Prozess zur Umsetzung von Ethylen zugeführt wird.

Vinylacetat wird in kontinuierlichen Verfahren unter Rückführung des aufgereinigten Produktstromes hergestellt. In einem heterogen katalysierten Gasphasenprozess reagiert Ethylen mit Essigsäure und Sauerstoff an Festbettkatalysatoren, welche im allgemeinen Palladium und Alkalimetallsalze auf einem Trägermaterial enthalten, und zusätzlich noch mit Gold, Rhodium oder Cadmium dotiert sein können.

Die Edukte Ethylen, Sauerstoff und Essigsäure werden in einer exothermen Reaktion im allgemeinen bei einem Druck von 8 bis 12 bar und einer Temperatur von 130°C bis 200°C in einem Festbettröhrenreaktor zu Vinylacetat umgesetzt:

C₂H₄ + CH₃COOH + 0.5 O₂ => CH₃COOCH=CH₂ + H₂O

Der Ethylenumsatz beträgt dabei etwa 10 %, der Essigsäureumsatz etwa 20 bis 30 % und der Sauerstoffumsatz bis zu 90 %.

Problematisch ist dabei, dass über die Edukte Ethylen und Sauerstoff Inertstoffe eingeschleust werden, welche schwer abtrennbar sind, aber bei Rückführung in den Reaktor und Anreicherung im Kreisgas die Selektivität der Reaktion zunehmend vermindern. Über den Sauerstoff werden die Inerten Stickstoff und Argon eingeschleppt, über Ethylen Ethan und in geringerem Ausmaß Methan. Diese Inerten müssen kontinuierlich aus dem System entfernt werden, sonst würde deren Anreicherung die Reaktion hemmen. Da Ethylen mit 60 bis 70 Vol-% den größten Teil des Kreisgasgemisches darstellt, geht die Inertenentfernung im allgemeinen mit einem deutlichen Ethylenverlust von etwa 1 bis 4 Vol-% der zugeführten Menge einher. Zusätzlich werden durch Nebenreaktionen Kohlendioxid und weitere Nebenprodukte wie Methylacetat und Ethylacetat gebildet.

Aufgrund des unvollständigen Umsatzes der Edukte wird in einer kontinuierlichen Prozessführung der gasförmige Produktstrom aufgearbeitet: In einem mit Essigsäure betriebenen Kreisgaswäscher wird das Zielprodukt Vinylacetat aus dem Kreisgas gewaschen und in nachfolgenden Destillationsprozessen aufgearbeitet. Das vinylacetatfreie Kreisgas wird über einen Kreisgasverdichter dem Essigsäuresättiger und anschließend dem Reaktor zugeführt. Um das Nebenprodukt CO₂ zu reduzieren wird ein Teil des vinylacetatfreien Kreisgases auf der Druckseite des Kreisgasverdichters ausgeschleust und einem Wasserwäscher zugeführt. Anschließend wird ein kleiner Anteil der Verbrennung zur Inertenausschleusung zugeführt, und der Restanteil in eine CO₂-Absorptionskolonne geleitet und dann dem Kreisgas CO₂-frei wieder zugeführt.

Die Inertenausschleusung mittels Entfernung von Ethylen aus dem Kreisgas verhindert eine Anreicherung von Ethan, Methan, Argon und Stickstoff im Kreisgasstrom. Die Menge des ausgeschleusten Inertenstroms wird in Abhängigkeit von der Ethylenkonzentration im Kreisgas geregelt. Werden zu geringe Mengen ausgeschleust, so werden die Inerten im Kreisgas aufkonzentriert und die Ethylenkonzentration im Kreisgas sinkt. Die Ethylenselektivität nimmt aber mit dem Ethylengehalt des Kreisgases zu. Je höher der Ethylengehalt im Kreisgas, das heißt je mehr Inerten-haltiges Ethylen aus dem Kreisgas entfernt wird und "frisches" Ethylen zugeführt wird, desto besser ist die Ethylenselektivität. Ab einem bestimmten Anteil aber ist eine weitergehende Ausschleusung von Inerten-haltigem Ethylen unwirtschaftlich, da jede zusätzliche Tonne Vinylacetatmonomer mit einem unverhältnismäßig hohem Anteil an ausgeschleustem Inerten-haltigem Ethylen erkauft werden muß. Da Ethylen teuer ist, steht die Rückgewinnung von Ethylen als kostensenkende Maßnahme an erster Stelle.

In der US 2003/0060642 A1 wird ein Verfahren zur Herstellung von Vinylacetat-Monomer beschrieben, bei dem ein Teil des Kreisgases ohne Inertenentfernung zurückgeführt wird, der Restanteil aber einer Purge Gas Treatment Unit und weiteren Einrichtungen zur Inertenentfernung, z. B. Demethanization zugeführt wird, und erst nach aufwendiger Reinigung teilweise zurückgeführt wird, wobei sogar noch ein Teilsstrom verbrannt wird .

In der GB 1264377 wird ein Verfahren zur Herstellung von Vinylacetat-Monomer mittels zweier Katalysatorkontakte beschrieben, bei dem in einem ersten Reaktor die Umsetzung von Ethylen, Essigsäure und Sauerstoff zu Vinylacetat stattfindet, der Produktstrom wird in einer Kolonne gereinigt und teilweise in den ersten Reaktor zurückgeführt. Der Restanteil wird einem zweiten Reaktor zugeführt zur nochmaligen Umsetzung zu Vinylacetat.

Gegenstand der WO 01/90042 A1 ist ein integrierter Prozess zur Herstellung von Vinylacetat mit vorgeschaltetem Schritt zur Herstellung von Essigsäure und Ethylen in einem Ethane Oxidation Reactor. Der ethylenhaltige Restgasstrom wird vor der Rückführung in den Ethane Oxidation Reactor einer Inertenentfernung unterzogen.

In der WO-A 01/00559 werden zwei gängigen Alternativen zur Ethylenrückgewinnung bei der Vinylacetat-Herstellung mittels Gasphasenreaktion von Ethylen, Essigsäure und Sauerstoff, beschrieben.
Dem aus dem Reaktor austretenden Gasstrom wird Kohlendioxid entzogen, und enthält dann primär Ethylen, Methan, Sauerstoff, Stickstoff und Argon. Bei Systemdruck wird der Gasstrom in eine Absorptionskolonne geleitet, mit Vinylacetat gewaschen, und am Kopf der Kolonne ein Gemisch aus Methan, Stickstoff, Sauerstoff und Argon abgezogen und der Verbrennung zugeführt. Am Boden der Kolonne werden Vinylacetat und Ethylen entnommen, das Gasgemisch entspannt und Ethylen von Vinylacetat abgetrennt. Das Ethylen wird anschließend komprimiert und in den Reaktor zurückgeleitet.
Nachteilig ist hierbei der energieaufwendige DekomprimierungsKomprimierungsschritt, sowie die Tatsache, dass sich die Inerten nicht vollständig entfernen lassen, und damit zunehmend angereichert werden und die Selektivität der Reaktion deutlich herabsetzen.

In einer weiteren Variante, auf die sich die WO-A 01/00559 richtet, wird der Großteil des gasförmigen Produktstroms bei Systemdruck mit Essigsäure in einem Absorptionsbehälter in Kontakt gebracht. Am Kopf der Kolonne werden Methan, Stickstoff, Sauerstoff und Argon abgetrennt und am Boden der Kolonne ein Gemisch aus Vinylacetat, Essigsäure und Ethylen abgezogen. Dieses Gemisch wird in einem Gaswäscher mit dem verbliebenen Anteil des gasförmigen Produktstroms in Kontakt gebracht. Das Ethylen wird am Kopf abgezogen und in den Reaktor zurückgeführt, das Vinylacetat wird am Boden der Kolonne gewonnen und der weiteren Aufarbeitung zugeführt.
Es entfällt der Dekomprimierungs/Komprimierungsschritt, aber auch hier werden die Inertgase zunehmend im Kreisgas angereichert.

Ein ähnliches Verfahren ist Gegenstand der US-A 3,714,237, wobei der gasförmige Produktstrom ebenfalls durch Wäsche mit Essigsäure aufgearbeitet wird, Vinylacetat abgetrennt wird, und das Restgas nach Auswaschen des Kohlendioxids in den Reaktor zurückgeführt wird. Auch hier werden die Inertgase zunehmend im Kreisgas angereichert.

Es bestand daher die Aufgabe den Kreisgasprozess bei der Herstellung von Vinylacetat so zu gestalten, dass die Anreicherung der genannten Inertgase weitestgehend unterbunden wird.

Gegenstand der Erfindung ist ein Verfahren zur Ethylenrückgewinnung in einem Kreisgasprozess zur Herstellung von Vinylacetat mittels
a) heterogen katalysierter Umsetzung von Ethylen, Essigsäure und Sauerstoff bei einem Druck von 1 bis 50 bar und einer Temperatur von 50°C bis 200°C,
b) Auftrennung des Produktgasstromes enthaltend im wesentlichen Ethylen, Vinylacetat, Essigsäure, Wasser, Kohlendioxid und weitere Inertgase, und
c) Rückführung von Ethylen in den Kreisgasprozess,
   dadurch gekennzeichnet, dass
d) der Produktgasstrom bei Systemdruck einem mit Essigsäure beschickten Kreisgaswäscher zugeführt wird, und Vinylacetat aus dem Kreisgas entfernt wird und,
e) das Vinylacetat-freie Kreisgas anschließend einer CO₂-Absorption zur Entfernung von Kohlendioxid zugeführt wird, und anschließend
f) ein Teil des ethylenhaltigen Kreisgastroms in das Reaktionssystem zurückgeführt wird, und 1 bis 25 Vol.-% des ethylenhaltigen Gasstromes ausgeschleust wird, und in Oxidationsverfahren zur Herstellung von Ethylenoxid und Ethylenglykol, von Acetaldehyd, in der Oxichlorierung von Ethylen zur Herstellung von Dichlorethan, in der Direktchlorierung von Ethylen zu Dichlorethan, in Verfahren zur Alkylierung von Benzol zu Ethylbenzol, zur Carbonylierung zu Acrylsäure, zur Polymerisation, in der Hydroformylierung zu Propionaldehyd, in der Reppe-Carbonylierung zu Propionsäure, oder bei der Alfolsynthese zur Herstellung von langkettigen, primären Alkoholen wieder verwendet wird.

Bei der kontinuierlichen Herstellung von Vinylacetat wird in Röhrenreaktoren gearbeitet, welche mit einem Festbettkatalysator beschickt sind. Diese Katalysatoren sind im allgemeinen mit Edelmetall(salz)en und Promotoren dotierte Trägerkatalysatoren, beispielsweise mit Palladium und mit Gold (Cadmiun) und K-Salzen dotierte Bentonit-Kugeln. Der Reaktor wird mit Ethylen, Sauerstoff und Essigsäure beschickt und die Reaktion vorzugsweise bei einem Druck von 8 bis 12 bar und einer Temperatur von 130°C bis 200°C durchgeführt. Der aus dem Reaktor austretende Produktgasstrom enthält im wesentlichen Vinylacetat, Ethylen, Essigsäure, Wasser, Sauerstoff, CO₂ sowie die Inerten Stickstoff, Argon, Methan und Ethan.

Der Produktgasstrom wird anschließend in einem mit Essigsäure betriebenen Kreisgas-Wäscher aufgetrennt, wobei Vinylacetat, Essigsäure, Wasser und weitere kondensierbare Anteile abgetrennt werden, und das Vinylacetat-Monomer mittels destillativer Aufbereitung gewonnen wird. Nach der Abtrennung der kondensierbaren Anteile (Vinylacetat, Essigsäure, Wasser) hat das Kreisgas üblicherweise folgende Zusammensetzung:
60 bis 65 Vol.-% Ethylen,
12 bis 18 Vol.-% CO₂
5 bis 8 Vol.-% Ethan,
4 bis 9 Vol.-% Sauerstoff,
4 bis 6 Vol.-% Stickstoff,
1 bis 2 Vol.-% Argon,
0.5 bis 1 Vol.-% Methan.
Diese Zusammensetzung macht deutlich, dass zur effektiven Entfernung von Methan, Ethan, Argon und Stickstoff, ein relativ hoher Anteil der Verbrennung zugeführt werden müßte, mit entsprechend hohem Ethylenverlust.

Bei der erfindungsgemäßen Vorgehensweise wird das Kreisgas nun in eine, üblicherweise mit wässriger Kaliumcarbonat-Lösung betriebene, CO₂-Absorption/Desorption geführt. Nach der CO₂-Wäsche hat das Kreisgas im allgemeinen folgende Zusammensetzung:
80 bis 83 Vol.-% Ethylen,
1 bis 4 Vol.-% CO₂,
2 bis 4 Vol.-% Ethan,
3 bis 5 Vol.-% Sauerstoff,
3 bis 4 Vol.-% Stickstoff,
0.5 bis 1 Vol.-% Argon,
0.2 bis 0.4 Vol.-% Methan.
Nach der CO₂-Wäsche wird der Produktstrom aufgeteilt. Ein Großteil des ethylenhaltigen Kreisgastroms wird über einen Kreisgasverdichter und Essigsäuresättiger in den Reaktor zurückgeführt. Der Rest des ethylenhaltigen Gasstromes wird ausgeschleust und in Verfahren zur Umsetzung von Ethylen wiederverwendet. Vorzugsweise werden 5 bis 20 Vol.-% des ethylenhaltigen Gasstromes ausgeschleust. Das mit Spuren von Kohlenwasserstoffen beladene Kohlendioxid wird zur thermischen Entsorgung geleitet.

Mit dem erfindungsgemäßen Verfahren ist mit der Inertenausschleusung nicht mehr zwangsläufig die Verbrennung des wertvollen Rohstoffes Ethylen verbunden. Es wird eine nahezu 100 %-ige Ausnutzung des Ethylens möglich; üblicherweise gehen durch die Inertenausschleusung 2 % des eingesetzten Ethylens verloren. Die Erzeugung von umweltbelastendem Kohlendioxid aufgrund Ethylenverbrennung wird vermieden. Dies entspricht einem Wegfall von 50 kg CO₂ pro Tonne Vinylacetatmonomer.

Die nachfolgenden Beispiele dienen zur weiteren Erläuterung der Erfindung:

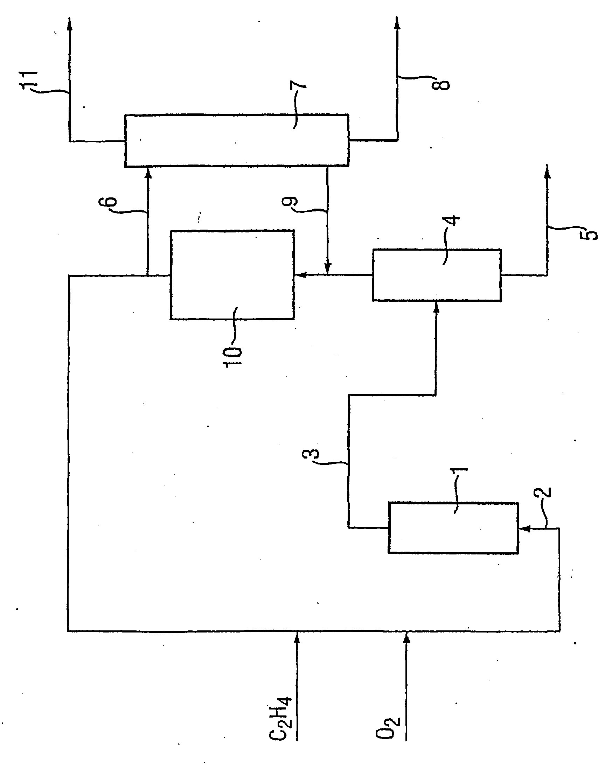

Figur 1 zeigt eine vereinfachte Darstellung des Prozesses:

### Beispiel 1:

Ein 25 m³ Röhrenreaktor 1 , welcher mit einem Pd/Au-Trägerkatalysator ausgerüstet war, wurde bei einem Druck von 8.5 bar und einer Temperatur von 160°C über Leitung 2 mit einem Gasgemisch mit einer GHSV von 3500 h-1 (Gas Hourly Space Velocity) beschickt. Das Gasgemisch (Kreisgas) hatte die nachfolgende Zusammensetzung:
61.7 Vol.-% Ethylen,
10.8 Vol.-% CO₂,
12.7 Vol.-% Essigsäure,
3.4 Vol.-% Ethan,
8.0 Vol.-% Sauerstoff,
0.8 Vol.-% Stickstoff,
0.8 Vol.-% Argon,
1.0 Vol.-% Methan
0.8 Vol.-% Wasser.

Das aus dem Reaktor 1 austretende Kreisgas wurde über Leitung 3 einem mit Essigsäure betriebenen Vinylacetat-Wäscher 4 zugeführt und anschließend über Leitung 6 einem mit Pottasche betriebenen Kohlendioxid-Wäscher 7 zugeführt. Über Leitung 5 wurde dem Vinylacetat-Wäscher 4 ein Vinylacetat/Essigsäure/Wasser-Gemisch entnommen und der weiteren Aufbereitung zugeführt. Nach der Kohlendioxid-Wäsche wurden 200 kg/h, das entspricht ca. 7 Vol.-%, des zur CO₂-Entfernung gehenden Kreisgases, über Leitung 8 zur Ethylenrückgewinnung in die Essigsäureherstellung verbracht, und der Rest über Leitung 9 und den Kreisgasverdichter 10 in den Reaktor 1 zurückgeführt. Die CO₂-Ausschleusung zur thermischen Entsorgung erfolgt über Weg 11.
Unter diesen Bedingungen zeigte der Katalysator eine Raum-Zeit-Ausbeute von 650 g/l·h bei einer Ethylenselektivität von 91.5 %.

### Beispiel 2:

Es wurde analog Beispiel 1 vorgegangen mit dem Unterschied, dass nach der CO₂-Wäsche 300 kg/h, das heißt ca. 10 Vol.-%, des zur CO₂-Entfernung gehenden Kreisgases, ausgeschleust worden sind. Dadurch erhöhte sich der Ethylen-Gehalt im Kreisgas auf 64 Vol.-%, die anderen Komponenten stellten sich entsprechend ein. Die Reaktionsbedingungen (GHSV, Kreisgasdruck etc.) blieben gleich.
Durch die Erhöhung der ausgeschleusten Menge stieg die Raum-Zeit-Ausbeute auf 660 g/l̇˙h bei einer Verbesserung der Ethylenselektivität auf 92.5 %, was einer mindestens 0.5 %-igen Steigerung der Vinylacetat-Produktion entspricht.

### Beispiel 3:

Es wurde analog Beispiel 1 vorgegangen mit dem Unterschied, dass nach der CO₂-Wäsche 450 kg/h, das heißt ca. 15 Vol.-%, des zur CO₂-Entfernung gehenden Kreisgases, zur Ethylenrückgewinnung in die Essigsäureherstellung verbracht worden sind. Dadurch erhöhte sich der Ethylen-Gehalt im Kreisgas auf 66 Vol.-%, die anderen Komponenten stellten sich entsprechend ein. Die Reaktionsbedingungen (GHSV, Kreisgasdruck etc.) blieben gleich.
Durch die Erhöhung der ausgeschleusten Menge stieg die Ethylenselektivität auf 93.0 %, was einer 1 %-igen Steigerung der Vinylacetat-Produktion entspricht.

## Patentansprüche

1. Verfahren zur Ethylenrückgewinnung in einem Kreisgasprozess zur Herstellung von Vinylacetat mittels
a) heterogen katalysierter Umsetzung von Ethylen, Essigsäure und Sauerstoff bei einem Druck von 1 bis 50 bar und einer Temperatur von 50°C bis 200°C,
b) Auftrennung des Produktgasstromes enthaltend im wesentlichen Ethylen, Vinylacetat, Essigsäure, Wasser, Kohlendioxid und weitere Inertgase, und
c) Rückführung von Ethylen in den Kreisgasprozess,
**dadurch gekennzeichnet, dass**
d) der Produktgasstrom bei Systemdruck einem mit Essigsäure beschickten Kreisgaswäscher zugeführt wird, und Vinylacetat aus dem Kreisgas entfernt wird und,
e) das Vinylacetat-freie Kreisgas anschließend einer CO₂-Absorption zur Entfernung von Kohlendioxid zugeführt wird, und anschließend
f) ein Teil des ethylenhaltigen Kreisgastroms in das Reaktionssystem zurückgeführt wird, und 1 bis 25 Vol.-% des ethylenhaltigen Gasstromes ausgeschleust wird, und in Oxidationsverfahren zur Herstellung von Ethylenoxid und Ethylenglykol, von Acetaldehyd, in der Oxychlorierung von Ethylen zur Herstellung von Dichlorethan, in der Direktchlorierung von Ethylen zu Dichlorethan, in Verfahren zur Alkylierung von Benzol zu Ethylbenzol, zur Carbonylierung zu Acrylsäure, zur Polymerisation, in der Hydroformylierung zu Propionaldehyd, in der Reppe-Carbonylierung zu Propionsäure, oder bei der Alfolsynthese zur Herstellung von langkettigen, primären Alkoholen wiederverwendet wird.

## Claims

1. Process for ethylene recovery in a cycle gas process for preparing vinyl acetate by means of
a) heterogeneously catalysed reaction of ethylene, acetic acid and oxygen at a pressure of from 1 to 50 bar and a temperature of from 50°C to 200°C,
b) separation of the product gas stream comprising substantially ethylene, vinyl acetate, acetic acid, water, carbon dioxide and further inert gases, and
c) recycling of ethylene into the cycle gas process,
**characterized in that**
d) the product gas stream is fed at system pressure to a cycle gas scrubber charged with acetic acid, and vinyl acetate is removed from the cycle gas, and
e) the vinyl acetate-free cycle gas is subsequently fed to a CO₂ absorption to remove carbon dioxide, and then
f) a portion of the ethylenic cycle gas stream is recycled into the reaction system, and from 1 to 25% by volume of the ethylenic gas stream is discharged and is reused in oxidation processes for preparing ethylene oxide and ethylene glycol, acetaldehyde, in the oxychlorination of ethylene for the preparation of dichloroethane, in the direct chlorination of ethylene to dichloroethane, in processes for alkylating benzene to ethylbenzene, for carbonylation to acrylic acid, for polymerization, in the hydroformylation to propionaldehyde, in the Reppe carbonylation to propionic acid, or in the Alfol process for preparing long-chain primary alcohols.

## Revendications

1. Procédé en vue de la récupération d'éthylène dans un processus de gaz en circuit en vue de la fabrication d'acétate de vinyle à l'aide
a) d'une conversion par catalyse hétérogène d'éthylène, d'acide acétique et d'oxygène à une pression de 1 à 50 bars et à une température de 50°C à 200°C,
b) d'une séparation du courant gazeux de produits contenant pour l'essentiel de l'éthylène, de l'acétate de vinyle, de l'acide acétique, de l'eau, du dioxyde de carbone et d'autres gaz inertes, et
c) d'une reconduite de l'éthylène dans le processus de gaz en circuit,
**caractérisé**
d) **en ce que** le courant gazeux de produits à la pression du système est acheminé à un laveur gazeux en circuit chargé d'acide acétique et en ce que l'acétate de vinyle est éliminé du gaz en circuit et
e) **en ce que** le gaz en circuit exempt d'acétate de vinyle est acheminé ensuite à une absorption de CO₂ en vue de l'élimination du dioxyde de carbone, et ensuite
f) **en ce qu'**une partie du courant gazeux en circuit contenant de l'éthylène est reconduit dans le système de réaction et en ce que de 1 à 25 % en volume du courant gazeux contenant de l'éthylène sont évacués et en ce qu'ils sont réutilisés dans les procédés d'oxydation en vue de la fabrication de l'oxyde d'éthylène et de l'éthylèneglycol, de l'acétaldéhyde, dans l'oxychloration de l'éthylène en vue de la fabrication de dichloroéthane, dans la chloration directe de l'éthylène pour former du dichloroéthane, dans des procédés en vue de l'alkylation du benzène pour former de l'éthylbenzène, en vue de la carbonylation pour former de l'acide acrylique, en vue de la polymérisation, dans l'hydroformylation pour former du propionaldéhyde, dans la carbonylation Reppe pour former de l'acide propionique ou lors de la synthèse d'alfols en vue de la fabrication d'alcools primaires à longue chaîne.
